(19)

Europäisches
Patentamt
European
Patent Office
Office européen
des brevets

(11) **EP 3 062 092 A1**

(12) **EUROPEAN PATENT APPLICATION**
published in accordance with Art. 153(4) EPC

(43) Date of publication:
**31.08.2016 Bulletin 2016/35**

(51) Int Cl.:
***G01N 23/04*** (2006.01)

(21) Application number: **14862920.7**

(22) Date of filing: **26.08.2014**

(86) International application number:
**PCT/JP2014/072318**

(87) International publication number:
**WO 2015/072206 (21.05.2015 Gazette 2015/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **14.11.2013 JP 2013236152**

(71) Applicant: **Sumitomo Rubber Industries, Ltd.**
**Kobe-shi, Hyogo 651-0072 (JP)**

(72) Inventors:
- **SAKAMAKI Ryuji**
  **Kobe-shi**
  **Hyogo 651-0072 (JP)**
- **ITO Wakana**
  **Kobe-shi**
  **Hyogo 651-0072 (JP)**
- **BITO Yasumasa**
  **Kobe-shi**
  **Hyogo 651-0072 (JP)**

(74) Representative: **Manitz, Finsterwald & Partner GbR**
**Martin-Greif-Strasse 1**
**80336 München (DE)**

(54) **POLYMER MATERIAL SIMULATION METHOD**

(57) To highly accurately represent behaviors of a polymer material when largely deformed. [ Solution ] A simulation method for a polymer material according to the present invention includes an imaging step S1 for acquiring electron beam transmission images of the polymer material 2, a step ₛ2 for constructing a three-dimensional image 21 of the polymer material, a model defining step S3 for defining a polymer material model 26, and a step S4 for carrying out a deformation simulation based on the polymer material model 26. The model defining step S3 includes a step S31 for constructing, based on the three-dimensional images 21 of the polymer material, a three-dimensional structure of the polymer material in which a filler portion 27 and a polymer material portion 28 are discriminated, a step S33 for disposing a filler model 35 in the filler portion 27, a step S34 for disposing a coarse-grained model 36 in the polymer material portion 28, and a step S37 for calculating structural relaxation based on a molecular dynamics calculation by the use of the filler model 35 and the coarse-grained model 36.

**FIG.9**

EP 3 062 092 A1

**Description**

**Technical field**

The present invention relates to a simulation method for calculating deformation of a polymer material containing a filler.

**Background art**

Polymer materials such as tire rubber contain a filler such as carbon black or silica for the purpose of reinforcement. It has been known that the dispersibility of a filler in a polymer material significantly affects the strength and the like of the rubber. But, the details are less well understood. For this reason, it is important to accurately observe a dispersion state of a filler in a polymer material, and to perform a simulation using a model based on the dispersion state.
In the following patent document 1, based on a three-dimensional structure of a polymer material constructed from electron beam transmission images, a polymer material model is defined. Therefore, in the patent document 1, the polymer material model may be defined based on a dispersion state of a filler.

Patent Document 1: Japanese Patent Application Publication No. 2013-57638

**Disclosure of the Invention**

**Problems that the Invention is to Solve**

In the patent document 1, the polymer material model is defined based on a finite element method. This polymer material model includes a filler model in which the filler is divided into a finite number of elements, and a polymer material model in which the polymer material is divided into a finite number of elements.
In the filler model and the polymer material model, the elements adjacent to each other share common nodes. Therefore, deformation of the filler model and the polymer material model is limited to a certain range. Accordingly, in the conventional simulation method, there is a problem such that the behavior of the polymer material deformed largely can not be expressed with a high degree of accuracy.
The present invention was made in view of the circumstances described above, and a main objective thereof is to provide a simulation method for a polymer material by which a behavior of the polymer material during large deformation can be expressed with a high degree of accuracy.

**Means for solving the Problems**

The present invention is a simulation method for calculating deformation of a polymer material containing a filler by the use of a computer, comprising
an imaging step of acquiring electron beam transmission images of the polymer material by the use of a scanning transmission electron microscope,
a step in which the computer constructs a three-dimensional image of the polymer material by a tomographic method based on the electron beam transmission images,
a model defining step in which the computer defines a polymer material model based on the three-dimensional image of the polymer material, and
a step in which the computer performs a deformation simulation based on the polymer material model, and characterized in that the model defining step compresses
a step of constructing a three-dimensional structure of the polymer material in which a filler portion where the filler is arranged and a polymer material portion around the filler portion are discriminated based on the three-dimensional image of the polymer material,
a filler model arranging step of arranging, in the filler portion, at least one filler model obtained by modeling the filler by using a plurality of filler particle models and a coupling chain model coupling between the adjacent filler particle models,
a coarse-grained model arranging step of arranging, in the polymer material portion, at least one coarse-grained model obtained by modeling a macromolecular chain of the polymer material by using a plurality of coarse-grained particle models and a coupling chain model coupling between the adjacent coarse-grained particle models, and
a step in which the computer calculates a structural relaxation based on a molecular dynamics calculation by using the filler model and the coarse-grained model.
It is preferable that the simulation method for a polymer material according to the present invention further comprises

a micro region selecting step in which the computer selects a micro region partitioned in the three-dimensional structure of the polymer material and having a predetermined size, and

in the filler model arranging step, the filler model is arranged in the filler portion in the micro region, and

in the coarse-grained model arranging step, the coarse-grained model is arranged in the polymer material portion in the micro region.

In the simulation method for a polymer material according to the present invention, it is preferable that the micro region selecting step comprises

a step of calculating the volume fraction of the filler portion in the three-dimensional structure of the polymer material,

a step of calculating the volume fraction of the filler portion in each micro region of a plurality of the micro regions partitioned at different positions in the three-dimensional structure of the polymer material,

a step of selecting, among a plurality of the micro regions, the micro region whose filler portion has the volume fraction mostly approximating the volume fraction of the filler portion in the three-dimensional structure of the polymer material.

In the simulation method for a polymer material according to the present invention, it is preferable that the filler particle models of the filler model are arranged in a face-centered cubic lattice.

In the simulation method for a polymer material according to the present invention, it is preferable that the coupling chain model of the filler model is defined according to a bond function or a particle distance restricting method.

### Effect of the invention

The simulation method for a polymer material according to the present invention includes

the imaging step of acquiring the electron beam transmission images of the polymer material by the use of the scanning transmission electron microscope,

the step of constructing the three-dimensional structure of the polymer material by the tomographic method based on the electron beam transmission images,

the model defining step of defining the polymer material model based on the three-dimensional structure of the polymer material, and

the step of performing the deformation simulation based on the polymer material model.

According to such method, it is possible to define an accurate polymer material model based on the actual polymer material.

The model defining step includes the step of constructing the three-dimensional structure of the polymer material in which the filler portion where the filler is arranged and the polymer material portion surrounding the filler portion are discriminated based on the three-dimensional image of the polymer material.

Further, the model defining step includes

the step of arranging, in the filler portion, at least one filler model obtained by modeling the filler by using a plurality of the filler particle models and the coupling chain model coupling between the adjacent filler particle models,

the step of arranging, in the polymer material portion, at least one coarse-grained model obtained by modeling the macromolecular chain of the polymer material by using the plurality of the coarse-grained particle models and the coupling chain model coupling between the adjacent coarse-grained particle models, and

the step in which the computer calculates the structural relaxation based on the molecular dynamics calculation by using the filler model and the coarse-grained model.

In such polymer material model, the filler models and the coarse-grained models are independently modeled based on the molecular dynamics method, differently from the finite element method in which the adjacent elements share common nodes. Therefore, in the simulation method for a polymer material of the present invention, behaviors of the polymer material when largely deformed can be expressed with a high degree of accuracy.

Moreover, in the model defining step, since the calculation of structural relaxation based on the molecular dynamics calculation is carried out, an equilibrium state of the filler model and the coarse-grained models can be calculated. By using such polymer material model, the simulation accuracy can be improved.

### Brief description of the drawings

[Figure 1] A perspective view of a computer in which the simulation method according to the present invention is executed.

[Figure 2] A schematic partial enlarged cross-sectional view of a polymer material.

[Figure 3] The structural expression of polybutadiene.

[Figure 4] A flow chart of an example of the processing procedure of the simulation method in the present embodiment.

[Figure 5] A schematic diagram showing an example of the scanning transmission electron microscope in the present embodiment.

[Figure 6] An illustration diagram showing a specimen when inclined.

[Figure 7] (a), (b) are side views showing a positional relationship between the focus and the sample in the imaging step.

[Figure 8] A perspective view showing the three-dimensional structure in the present embodiment.

[Figure 9] A flowchart showing an example of the processing procedure of the model defining step.

[Figure 10] A perspective view conceptually showing a virtual space.

[Figure 11] A conceptual diagram showing the filler model.

[Figure 12] A conceptual diagram showing the filler particle models and coupling chain models.

[Figure 13] A conceptual diagram showing the coarse-grained model.

[Figure 14] A conceptual diagram showing an enlarged view of the filler model and the coarse-grained model.

[Figure 15] A flowchart showing an example of the processing procedure of the micro region selecting step in another embodiment of the present invention.

[Figure 16] A flowchart showing an example of the processing procedure of a micro region volume fraction calculation step.

[Figure 17] A perspective view showing a micro region in the three-dimensional structure.

## Description of the signs

21    three-dimensional structure
26    polymer material model
27    filler portion
28    polymer material portion
35    filler model
36    coarse-grained model

## Best mode for Carrying out the Invention

Hereinafter, an embodiment of the present invention will be described with reference to the accompanying drawings.

In the simulation method for a polymer material according to the present embodiment (hereinafter, simply referred to as "simulation method"), deformations of the polymer material containing a filler are calculated by the use of a computer.

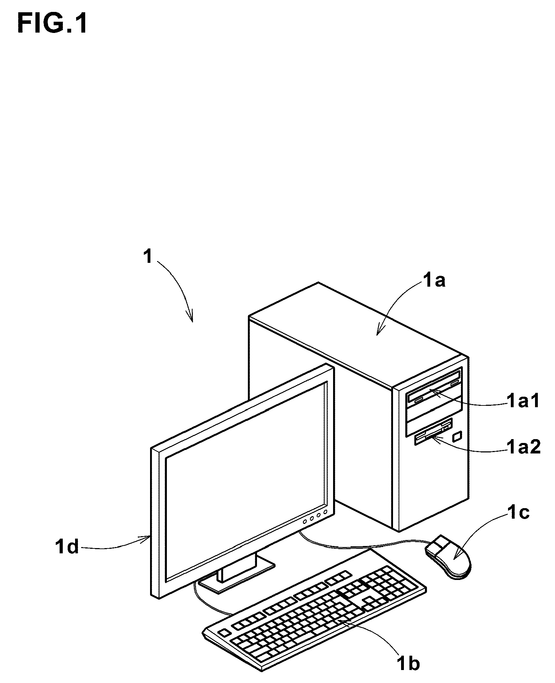

Figure 1 is a perspective view of the computer for executing the simulation method of the present invention. The computer 1 includes a body 1a, a keyboard 1 b, a mouse 1c, and a display device 1d. This body 1a is, for example, provided with an arithmetic processing unit (CPU), ROM, a working memory, a storage device such as a magnetic disk, and the disk drive apparatus 1a1, 1a2. In the storage device, software for performing the simulation method in the present embodiment, etc. are stored in advance.

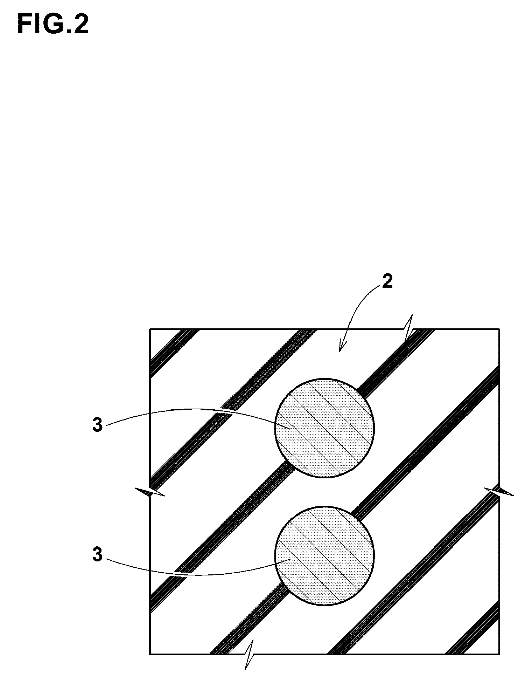

Figure 2 is a schematic partial enlarged sectional view of a polymer material in the present embodiment.

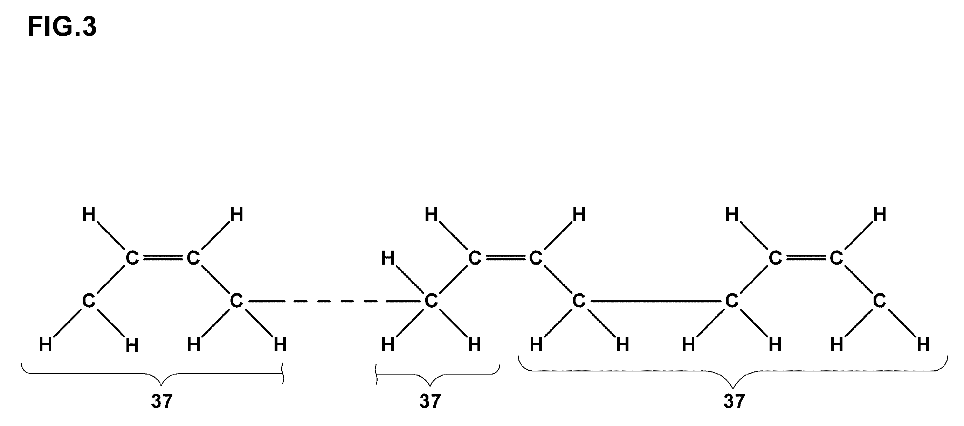

Figure 3 is a structural expression of polybutadiene. As the polymer material 2, for example, rubber, resin, or elastomer is included.

As the polymer material 2 in the present embodiment, cis-1,4-polybutadiene (hereinafter, simply referred to as "polybutadiene") is taken for instance.

A macromolecular chain constituting the polybutadiene is constructed by connecting, with a polymerization degree n, monomers {- [$CH_2$-CH=CH-$CH_2$] -} constructed by methylene groups (-$CH_2$-) and methine groups (-CH-).

Incidentally, a polymer material other than polybutadiene may be used as the polymer material.

As the filler 3 contained in the polymer material 2, for example, carbon black, silica, or alumina is included.

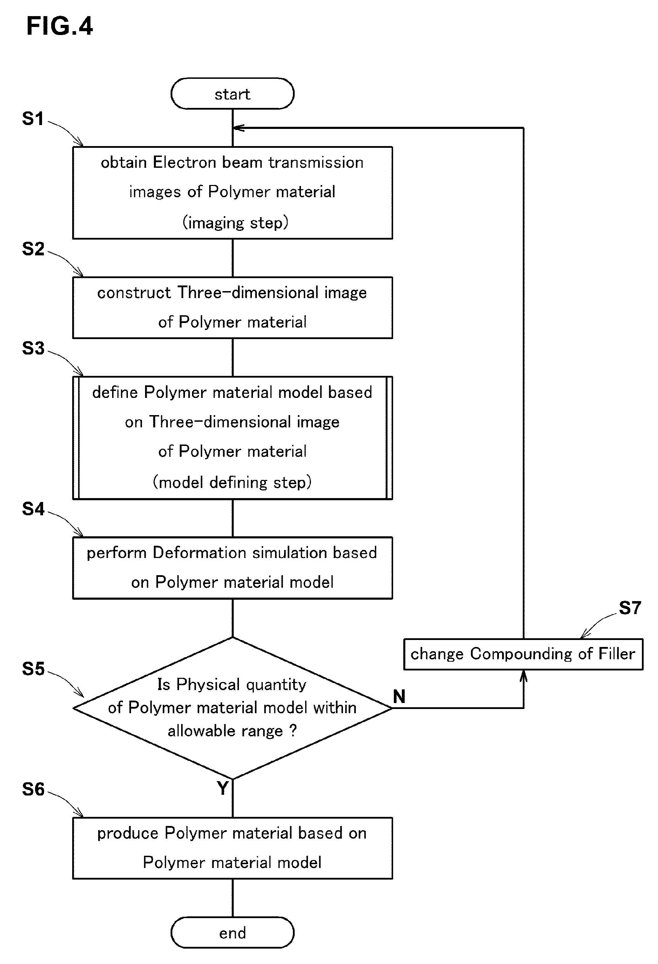

Figure 4 is a flowchart showing an example of the processing procedure of the simulation method in the present embodiment.

In the simulation method in the present embodiment, first, by using a scanning transmission electron microscope, electron beam transmission images of the polymer material 2 are obtained (imaging step S1).

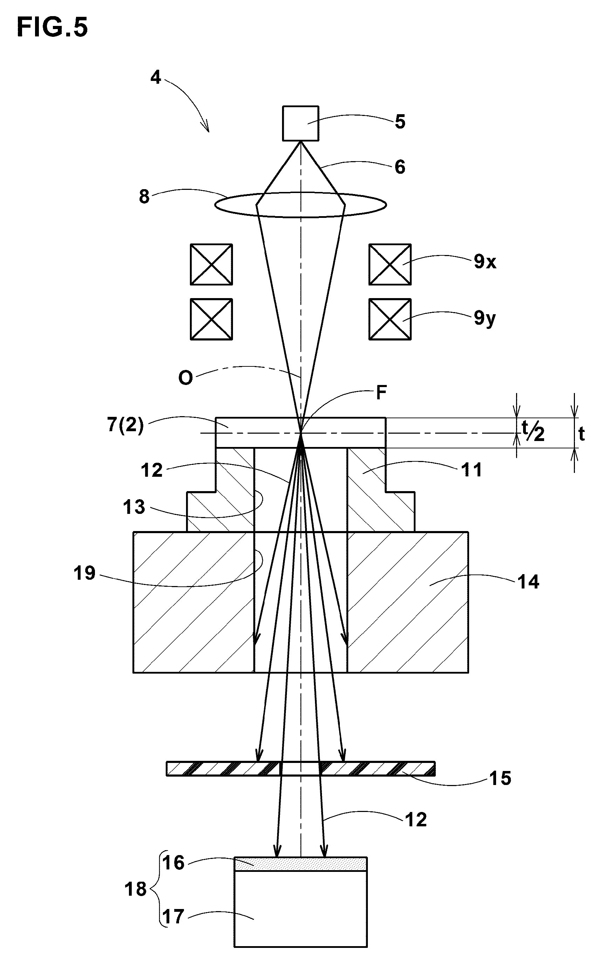

Figure 5 is a schematic diagram showing an example of the scanning transmission electron microscope in the present embodiment.

Similarly to the conventional scanning transmission electron microscope, the scanning transmitted electrons microscope device 4 is configured to include an electron gun 5, a focusing lens 8, an X-direction sweeping coil 9x and a Y-direction sweeping coil 9y.

The focusing lens 8 is for focusing on a sample 7 of the polymer material 2 a primary electron beam 6 emitted from the electron gun 5 downwardly and perpendicularly to the horizontal plane. The X-direction sweeping coil 9x and the Y-direction sweeping coil 9y are for sweeping the primary electron beam 6 on the sample 7 in the X-direction and the Y-direction. The sample 7 is formed in a plate shape having a constant thickness t.

The sample 7 is fixed to a sample holder 11. A central portion of the sample holder 11 is provided with an electron beam passing hole 13 penetrating along the axis O of lens for the electron beam. The transmitted electrons 12 through the sample 7 passes the electron beam passing hole 13. The sample holder 11 is attached to a sample stage 14. A central portion of the sample stage 14 is provided with an electron beam passing hole 19 penetrating along the axis O of lens for the electron beam. The electron beam passing hole 19 and the electron beam passing hole 13 are continuous along the axis O of lens for the electron beam. Further, on the downstream side of the sample stage 14, the scattering angle limiting aperture 15 for limiting the passage of transmitted electrons 12 is provided.

On the downstream side of the scattering angle limiting aperture 15, there are disposed a scintillator 16 for converting the transmitted electrons 12 to light, and a photomultiplier tube 17 which converts the converted light into an electronic signal. The scintillator 16 and the photomultiplier tube 17 constitute a detector 18 for the transmitted electrons. Incidentally, the sample stage 14, the scattering angle limiting aperture 15, the scintillator 16 and the photomultiplier tube 17 are disposed within a specimen chamber (not shown) of the scanning transmission electron microscope device 4. Further, the scanning transmission electron microscope device 4 is provided with a specimen inclining unit (not shown) for inclining (rotating) the sample 7 with respect to the electron beam.

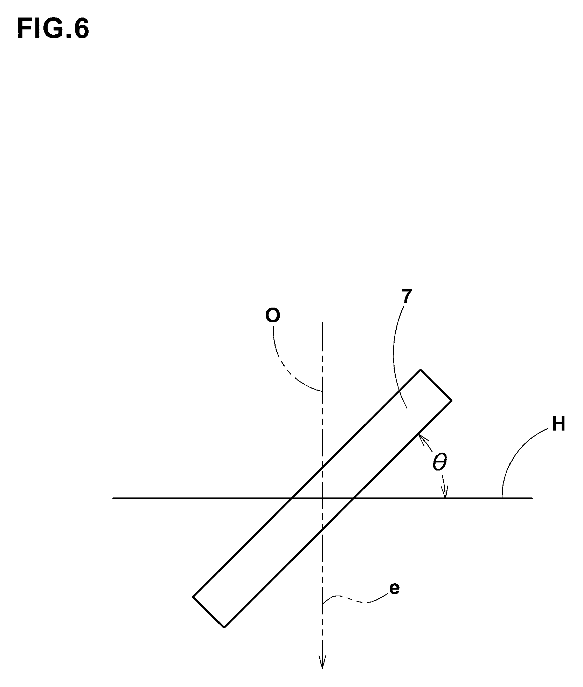

Figure 6 is an explanatory view showing a state in which the sample 7 is inclined.

The specimen inclining unit can hold the specimen 7 inclining at an angle $\theta$ ($\theta$ not equal to 0) with respect to the horizontal plane H. Thus, the specimen inclining unit serves to image the sample 7 in a plurality of angle states having different angles with respect to the axis O of lens for the electron beam e.

In the imaging process s1 using such scanning transmission electron microscope, first, as shown in Figure 5, the sample holder 11 to which the sample 7 is fixed, is mounted on the sample stage 14 by an operator. Then, the primary electron beam 6 emitted from the electron gun 5 is focused by the focusing lens 8 and swept on the sample 7 by the x-direction, Y-direction sweeping coils 9x, 9y. By such scanning of the sample 7 with the primary electron beam 6, the transmitted electrons 12 scattered in the sample 7 and penetrating therethrough, or, the transmitted electrons 12 not scattered in the specimen 7 and penetrating therethrough exits from the lower surface of the sample 7.

The transmitted electrons 12 emitted from the lower surface of the sample 7 reach the scattering angle limiting aperture 15 after passing through the electron beam passing hole 13 of the sample holder 11 and the electron beam passing hole 19 of the sample stage 14. The transmitted electrons 12 having a particular scattering angle pass through the scattering angle limiting aperture 15, and collide against the scintillator 16 so as to be converted into light, and then converted into an electric signal by the photomultiplier tube 17. This electrical signal is sent to a display means via an A/D converter (both not shown). In the display means, the signal sent is intensity modulated, and an electron beam transmission image that reflects the internal structure of the sample 7 is displayed. Thus, a plurality of images corresponding to the scanning position can be acquired.

Further, in the imaging step s1, as shown in Figure 6, the sample 7 is inclined (rotated) by the specimen inclining unit (not shown). Then, the sample 7 is imaged under a plurality of angle states having different angles with respect to the axis O of lens for the electron beam e. In the present embodiment, the sample 7 is inclined from a measurement start angle to a measurement end angle at a predetermined angle unit. And, at every angle, the acquisition of the electron beam transmission image is repeated. Thus, in the imaging step s1, rotation series images (a plurality of electron beam transmission images) are obtained. Such rotation series images are stored in the computer 1.

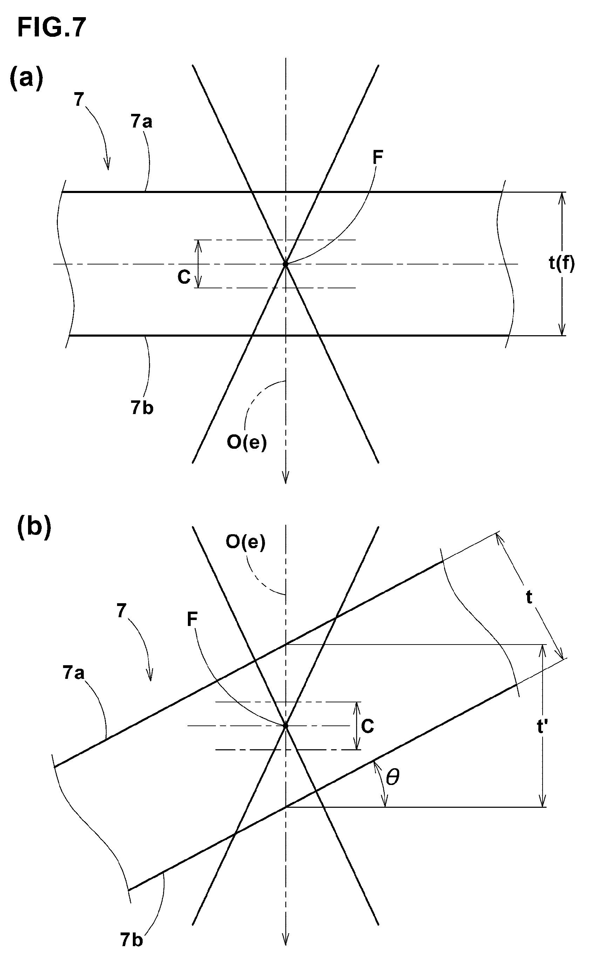

Figure 7 (a), (b) is side views showing the positional relationship between the focus F and the sample 7 in the imaging step S1.

It is desirable that the focal point F of the scanning transmission electron microscope device 4 is adjusted to a central region C of the thickness t of the sample 7 (rubber material) at a plurality of the angle states having different angles with respect to the optical axis O of the electron beam. Thus, a range in which a sharp image is obtained, i.e., the range of the focal depth f can be ensured widely within the interior of the sample 7.

It is preferable that the central region C is a region of not more than 30% of the thickness t from the central position of the thickness t of the sample 7.

As shown in Figure 7 (b), when the upper surface 7a and the lower surface 7b of the sample 7 are not orthogonal to the axis O of lens for the electron beam e, it is preferably defined by an apparent thickness t' (ie, t/cos $\theta$) along the optic axis of the electron beam e across the sample 7.

Next, as shown in Figure 4, the computer 1 constructs a three-dimensional image of the polymer material 2 by a tomographic method based on the electron beam transmission images (step S2).

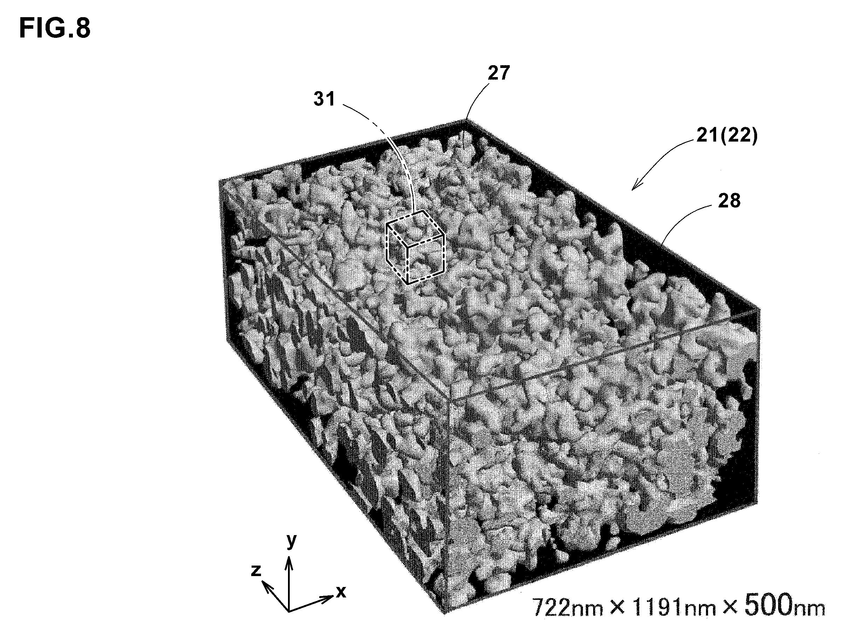

Figure 8 is a perspective view showing a three-dimensional image (three-dimensional structure) in the present embodiment.

In the step S2, based on the tomographic method, a plurality of the electron beam transmission images acquired at the respective angles are reconstructed as a three-dimensional image of the polymer material (hereinafter, simply referred to as "three-dimensional image") 21 as shown in Figure 8. In such three-dimensional image 21, a dispersion state of the filler 3 in the polymer material 2 (shown in Figure 2) is clearly shown in three dimensions. Such three-dimensional image 21 is stored in the computer 1.

Next, as shown in Figure 4, the computer 1 defines a polymer material model based on the three-dimensional image 21 of the polymer material (model defining step s3).

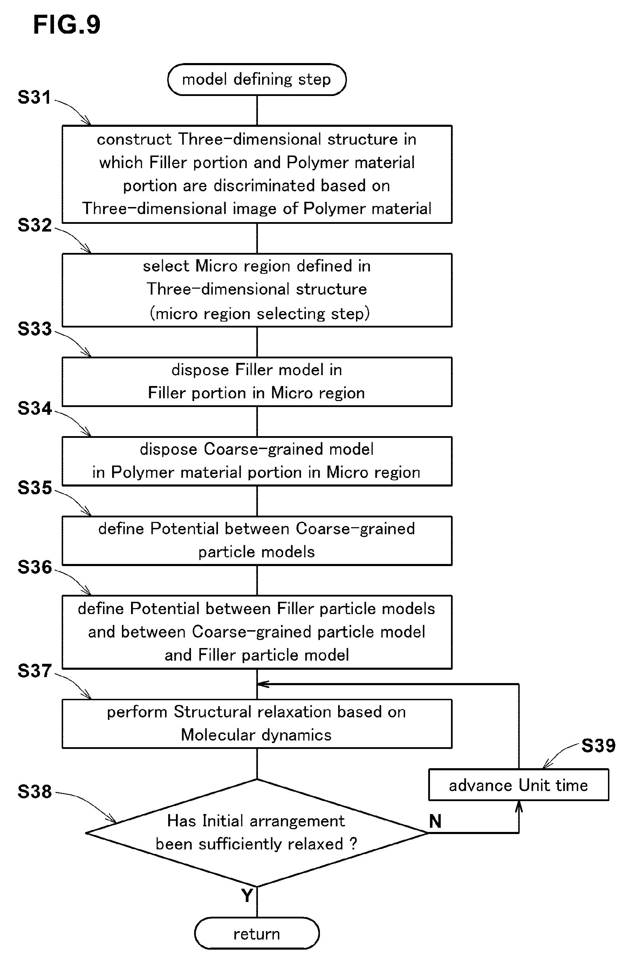

Figure 9 is a flowchart showing an example of the processing procedure of the model defining step s3.

In the model defining step s3 in the present embodiment, first, a three-dimensional structure of the polymer material (hereinafter, simply referred to as "three-dimensional structure") 22, in which a filler portion 27 (shown in Figure 2) where the filler 3 was arranged and a polymer material portion 28 around the filler portion 27 are discriminated, is constructed, based on the three-dimensional image 21 shown in Figure 8 (step s31).

In the step S31, first, positions of cross sections are specified on the three-dimensional image 21, and a plurality of two-dimensional slice images are acquired.

Next, by image processing, each slice image is divided into at least two portions of the filler portion 27 and the polymer material portion 28.

In the image processing, first, a threshold value for data such as brightness or luminosity of the image is set in advance. Next, based on the set threshold, a slice image is automatically discriminated into the filler portion 27 and the polymer material portion 28.

Then, based on a plurality of the slice images that have been discriminated, a three-dimensional structure 22 (shown in Figure 8) in which the filler portion 27 and the polymer material portion 28 are discriminated, is constructed.

The three-dimensional structure 22 is image data. The three-dimensional structure 22 is stored in the computer 1.

Next, the computer 1 selects a micro region 31 defined in the three-dimensional structure 22 (micro region selection step S32).

The micro region 31 has a predetermined size.

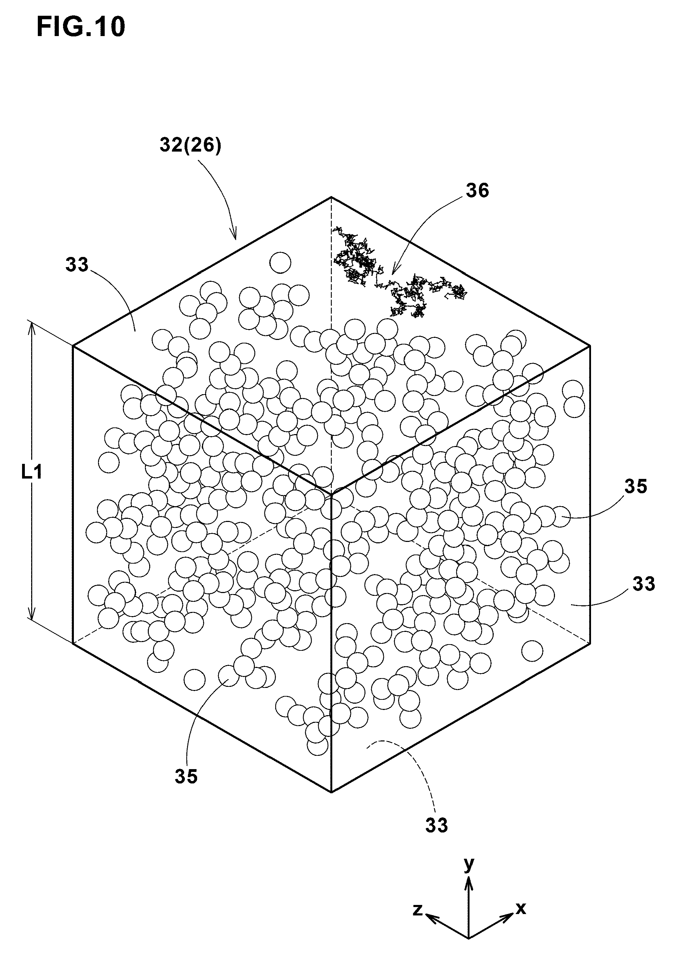

The size of the micro region 31 is the same as the size of a virtual space 32 (shown in Figure 10) which is an object of calculation in the after-mentioned molecular dynamics simulation. Thus, in the after-mentioned simulation, since the object of calculation is limited to the range of the micro region 31, the computational time can be shortened.

Incidentally, the micro regions 31 may be defined at an arbitrary position in the three-dimensional structure 22. Such micro region 31 is stored in the computer 1.

Figure 10 is a perspective view conceptually showing the virtual space 32.

For example, the virtual space 32 in the present embodiment is defined as a cube having at least one pair, in the present embodiment, three pairs of opposite surfaces 33 and 33. In the interior of the virtual space 32, a plurality of the after-mentioned filler models 35 and coarse-grained models 36 are disposed.

It is preferable that the distance between the paired surfaces 33, 33 (ie, the length L1 of one side) is, for example, set to be 50nm to 1000nm ($76\sigma$ to $1515\sigma$ in the unit of the molecular dynamics calculation).

such virtual space 32 is stored in the computer 1.

Then, in the filler portion 27 in the micro region 31, there is disposed at least one filler model obtained by modeling the filler 3 (shown in Figure 2) (step S33).

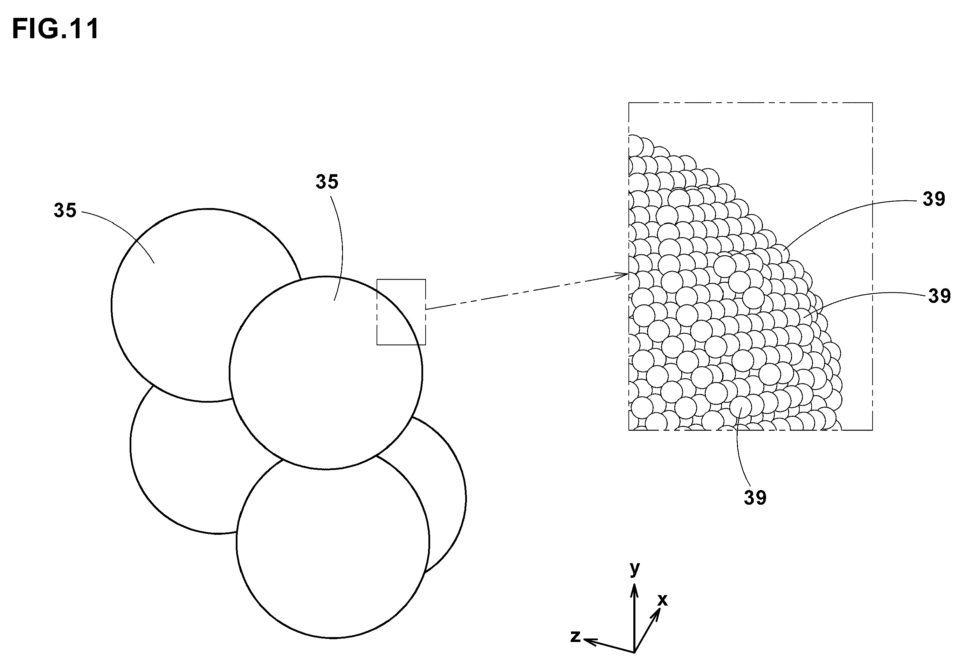

Figure 11 is a conceptual diagram of the filler model 35.

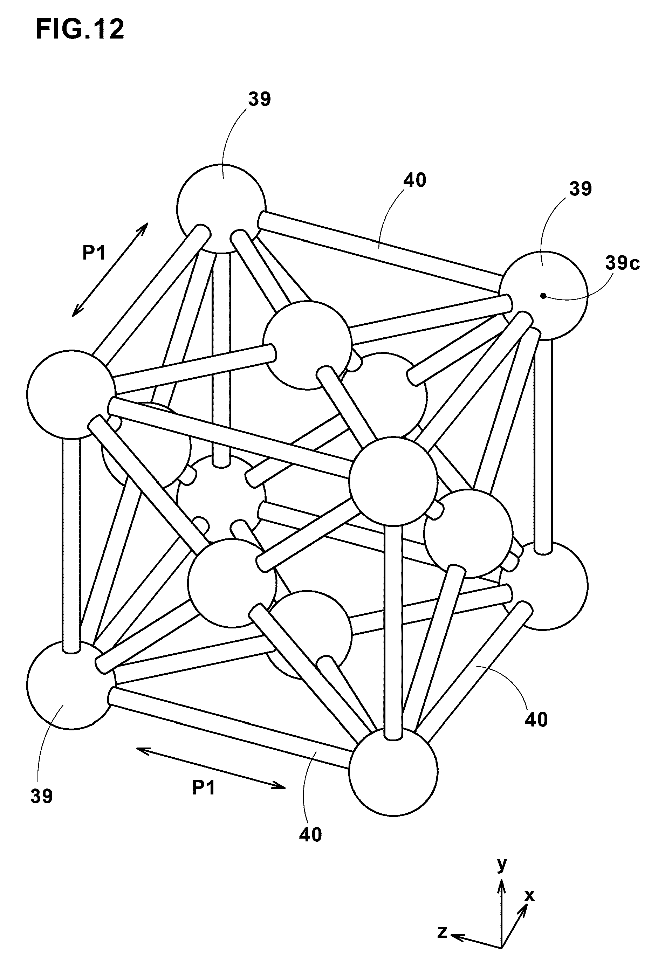

Figure 12 is a conceptual diagram showing the filler particle models 39 and coupling chain models 40.

The filler model 35 includes a plurality of filler particle models 39 and the coupling chain model 40 binding between the adjacent filler particle models 39, 39.

The filler particle model 39 is treated as a material point of the motion equation in the molecular dynamics calculation. That is, on the filler particle model 39, there are defined parameters such as mass, volume, diameter, charge or initial coordinates.

In the step S33 in the present embodiment, first, as shown in Figure 10, in the computer 1, the image data (not shown) of the micro region 31 (shown in Figure 8) is superimposed upon the virtual space 32.

Next, a plurality of the filler particle models 39 (shown in Figure 11) are arranged in the region of the filler portion 27 represented in the virtual space 32 (shown in Figure 8).

It is desirable that the filler particle models 39 are arranged in a face-centered cubic lattice as shown in Figure 12. Thus, as the filler particle models 39 are coupled in a crystal lattice, movements of the filler particle models 39 can be firmly restrained, therefore, the filler model 35 (shown in Figure 11) can be provided with higher rigidity. In the after-mentioned molecular dynamics calculation, such filler model 35 can approximate physical properties of the filler 3 (shown in Figure 2).

Incidentally, the filler particle models 39 may be arranged, for example, in a body-centered cubic lattice, or in a crystal lattice such as simple lattice.

Next, in the step S33, the coupling chain model 40 is defined.

The coupling chain model 40 in the present embodiment is defined based on a bond function. Namely, the coupling chain model 40 is defined by a potential P1 represented by the sum of a potential defined by the following expression (1) (hereinafter, may be referred to as "LJ potential $U_{LJ}(r_{ij})$)" and a bonding potential $U_{FENE}$ defined by the following expression (2).

[Expression 1]

$$U_{LJ}(r_{ij}) = \begin{cases} 4\varepsilon\left[\left(\dfrac{\sigma}{r_{ij}}\right)^{12} - \left(\dfrac{\sigma}{r_{ij}}\right)^{6}\right], & r_{ij} < r_c \\ 0, & r_{ij} \geq r_c \end{cases} \quad ...(1)$$

[Expression 2]

$$U_{FENE} = \begin{cases} -0.5kR_0^{\,2}\ln\left[1 - \left(\dfrac{r_{ij}}{R_0}\right)^{2}\right], & r_{ij} < R_0 \\ \infty, & r_{ij} \geq R_0 \end{cases} \quad ...(2)$$

Here, constants and variables are parameters of the respective potentials of the Lennard-Jones and FENE as follows.

$r_{ij}$: distance between particles
$r_C$: cut-off distance
k: spring constant between the particles
$\in$: the intensity of the LJ potential defined between the particles
$\sigma$: corresponds to the diameter of the particles
$R_0$ : fully-stretched length

The distance $r_{ij}$, cut-off distance $r_C$ and fully-stretched length $R_0$ are defined as the distance between the centers 39c of the filler particle models 39.

In the above expression (1), when the distance $r_{ij}$ between the filler particle models 39, 39 becomes smaller, the LJ potential $U_{LJ}(r_{ij})$ which exerts a repulsive force is increased.

In the above expression (2), on the other hand, when the distance $r_{ij}$ between the filler particle models 39, 39 becomes larger, the bonding potential $U_{FENE}$ which exerts an attractive force is increased.

Therefore, the potential P1 defines a restoring force for returning the distance $r_{ij}$ to a position at which the LJ potential $U_{LJ}(r_{ij})$ and the bonding potential $U_{FENE}$ are balanced each other.

Further, in the above expression (1), when the distance $r_{ij}$ between the filler particle model 39, 39 becomes smaller, the LJ potential $U_{LJ}(r_{ij})$ becomes infinitely larger. On the other hand, in the above expression (2), when the distance $r_{ij}$ becomes equal to or more than the fully-stretched length $R_0$, the bonding potential $U_{FENE}$ is set to infinity. Therefore, in the potential P1, the distances $r_{ij}$ equal to or more than the fully-stretched length $R_0$ is not allowed.

Incidentally, the intensity $\in$ of each potential of the LJ potential $U_{LJ}(r_{ij})$ and FENE, the fully-stretched length $R_0$, the diameter $\sigma$ of the particle, the cut-off distance $r_C$ can be set arbitrarily.

It is preferable that these constants are set as follows, for example, based on Article 1 (Kurt Kremer & Gary S. Grest "Dynamics of entangled linear polymer melts: A molecular-dynamics simulation ", J Chem Phys vol.92, No.8, 15 April 1990)

intensity $\in$: 1.0
fully-stretched length $R_0$ : 1.5
distance $\sigma$: 1.0
cut-off distance $r_C$ : $2^{1/6}\sigma$

A spring constant k is a parameter which determines the rigidity of the filler model 35 (shown in Figure 11). Therefore, it is preferable that the spring constant k is set in a range from 10 to 5,000 based on the rigidity of the filler 3. Incidentally, when the spring constant k is smaller than 10, the rigidity of the filler model 35 becomes excessively small, and simulation accuracy may be degraded.

Conversely, if the spring constant k becomes greater than 5000, deformation of the filler model 35 is substantially not allowed, and the molecular dynamics calculation may become unstable. From these points of view, the spring constant k is more preferably 20 or more, still more preferably 25 or more, and more preferably 3000 or less, still more preferably 2500 or less.

since such coupling chain model 40 is defined, the rigidity of the filler model 35 (shown in Figure 11) is increased. Thereby, the filler model 35 approximate to the filler 3 (shown in Figure 2) can be defined in the after-mentioned molecular dynamics simulation.

Since the filler particle model 39 and the coupling chain model 40 are sequentially modeled as above, the filler model 35 shown in Figure 10 is defined.

In the present embodiment, since the filler model 35 is defined based on the filler portion 27 (shown in Figure 8) discriminated from the actual polymer material 2, the accurate polymer material model 26 can be defined.

such filler model 35 is stored in the computer 1.

In this embodiment, the coupling chain model 40 is defined based on the bond function, but it is not limited thereto. For example, the coupling chain model 40 may be defined based on the particle distance restricting method.

For example, the SHAKE method can be employed as the particle distance restricting method. In the SHAKE method, the binding force of the filler particle models 39, 39 is derived based on the method of Lagrange multiplier. Thus, in the coupling chain model 40 defined by the SHAKE method, the distance between the particles is fixed to a constant value.

On the other hand, in the coupling chain model 40 defined by the bonding function, the distance between the particles varies fast in the vicinity of the equilibrium length. Therefore, when the coupling chain model 40 defined by the bonding function is compared with the coupling chain model 40 defined by the SHAKE method, even if a larger unit time is defined in the after-mentioned molecular dynamics calculation, the calculation becomes stable.

Next, as shown in Figure 10, at least one coarse-grained model 36 in which the polymer chains of the polymer material 2 has been modeled is arranged in the polymer material portion 28 (shown in Figure 8) in the micro region 31 (step S34).

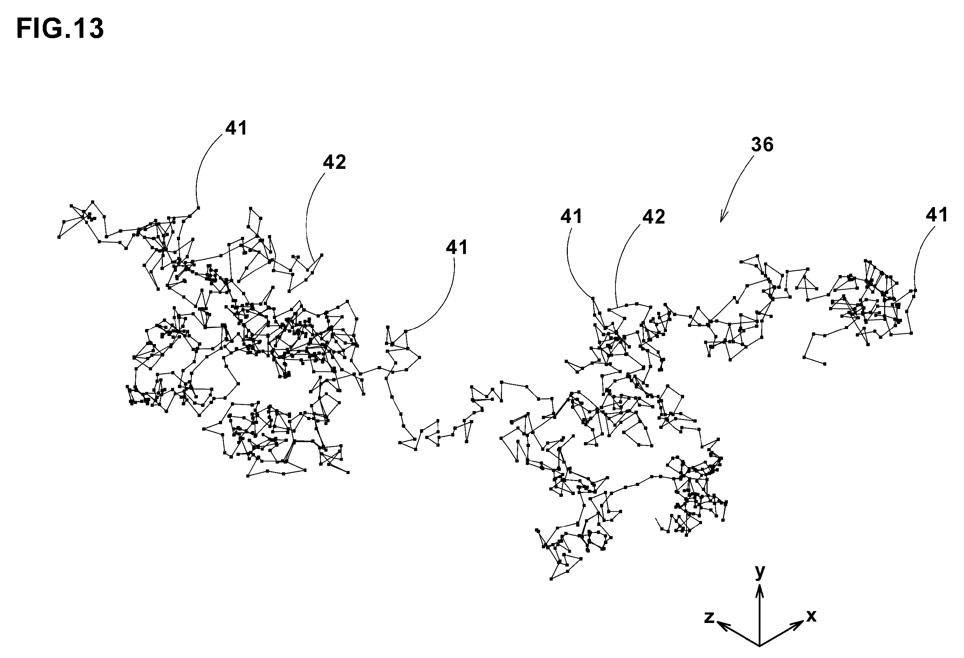

Figure 13 is a conceptual diagram showing a coarse-grained model 36.

Each coarse-grained model 36 is configured to include a plurality of coarse-grained particle models 41, and a coupling chain model 42 which couples between the adjacent coarse-grained particle models 41.

The coarse-grained particle model 41 is one in which a structural unit constituting a part of the monomer or monomers of the polymer material 2 (FIG. 2) was replaced by a single particle.

As shown in Figures 2 and 13, when the macromolecular chain of the polymer material 2 is polybutadiene, for example, supposing 1.55 pieces of monomers as a structural unit 37, the structural unit 37 is replaced by one coarse-grained particle model 41. Thereby, a plurality of (for example, 10 to 5000) coarse-grained particle models 41 are defined in the coarse-grained particle model 41.

The reason why 1.55 pieces of monomers were supposed as a structural unit 37, is based on the descriptions of the above-mentioned Article 1 and the above-mentioned Article 2 (L,J.Fetters, D.J.Lohse and R.H.Colby, "Chain Dimension and Entanglement Spacings" Physical Properties of Polymers Handbook Second Edition P448).

If the polymer chain is other than polybutadiene, the structural unit 37 may be defined, for example, based on the above-mentioned Articles 1 and 2.

The coarse-grained particle model 41 is treated as a material point of the motion equation in the molecular dynamics calculation. Namely, on the coarse-grained particle model 41, parameters, for example, mass, volume, diameter or charge are defined.

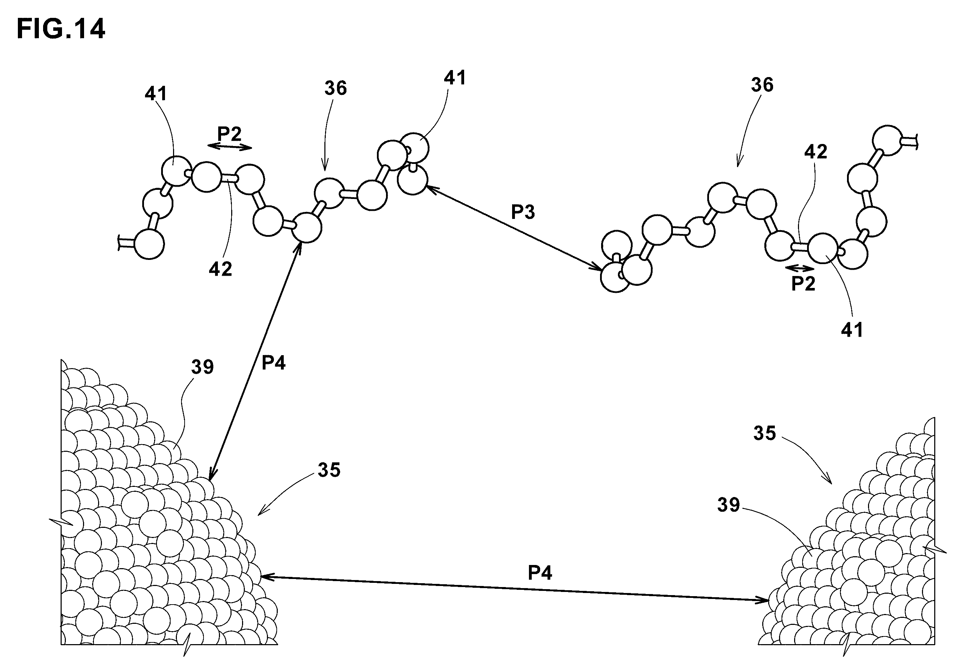

Figure 14 is a conceptual diagram showing an enlarged view of the filler model 35 and the coarse-grained model 36. The coupling chain model 42 is defined by a potential P2 in which a fully-stretched length is defined between the coarse-grained particle models 41, 41.

The potential P2 in this embodiment is defined by the sum of the LJ potential $U_{LJ}(r_{ij})$ defined by the above expression (1) and the bonding potential $U_{FENE}$ defined by the expression (2). The values of the constants and the values of the variables of the LJ potential $U_{LJ}(r_{ij})$ and the bonding potential $U_{FENE}$ can be set arbitrarily.

In the present embodiment, based on the above-mentioned Article 1, the following values are set.

Spring constant k: 30
fully-stretched length $R_0$: 1.5
intensity $\epsilon$: 1.0
distance $\sigma$: 1.0
cut-off distance $r_C$ : $2^{1/6} \sigma$

Thereby, it is possible to define the coarse-grained model 36 in the form of a straight-chain in which the adjacent coarse-grained particle models 41, 41 are bounded stretchably by such coupling chain models 42.

Since the coarse-grained particle models 41 and the coupling chain model 42 are sequentially modeled as above, the coarse-grained model 36 is defined.

Then, in the step S34 in the present embodiment, in the virtual space 32 (shown in Figure 10) upon which the image data (not shown) of the micro region 31 (shown in Figure 8) is superimposed in the computer 1, a plurality (e.g., 10 to 1,000,000) of the coarse-grained models 36 are arranged in the polymer material portion 28 (shown in Figure 8) presented in the virtual space 32.

since the coarse-grained model 36 is defined based on the polymer material portion 28 discriminated from the actual polymer material 2, the accurate polymer material model 26 can be defined.

These coarse-grained models 36 are stored in the computer 1.

Then, a potential P3 is defined between the coarse-grained particle models 41, 41 of the adjacent coarse-grained models 36, 36 (step S35).

The potential P3 is defined by the LJ potential $U_{LJ}(r_{ij})$ of the expression (1).

Incidentally, the intensity $\in$ and constant $\sigma$ of the potential P3 can be set arbitrarily.

It is preferable that the intensity $\in$ and constant $\sigma$ of the potential P3 in the present embodiment are set in the same ranges as the intensity $\in$ and constant $\sigma$ of the potential P2 of the coupling chain model 42.

The potential P3 is stored in the computer 1.

Then, a potential P4 is defined between the filler particle models 39, 39 of the adjacent filler models 35, and, between the coarse-grained particle model 41 and the filler particle model 39 (step S36).

The potential P4 is defined by the LJ potential $U_{LJ}(r_{ij})$ of the expression (1).

Incidentally, the values of the constants and variables of the potential P4 can be set arbitrarily.

It is preferable that the constants and variables of the potential P4 in the present embodiment are defined based on the above-mentioned Article 1.

The potential P4 is stored in the computer 1.

Next, using the coarse-grained model 36 and the filler model 35 shown in Figure 10, the computer 1 calculates the structural relaxation based on the molecular dynamics calculation (step S37).

In the molecular dynamics calculation in the present embodiment, for example, the Newton's motion equation is applied to the virtual space 32, supposing that the filler model 35 and the coarse-grained model 36 accord with the classical dynamics in a predetermined period of time.

And motions of the filler model 35 and the coarse-grained model 36 at respective time points are tracked every unit time.

In the present embodiment, when calculating the structural relaxation, in the virtual space 32, the pressure and temperature are kept constant, or the volume and temperature are kept constant. Thereby, in the step S37, the initial arrangement of the filler model 35 and the coarse-grained model 36 can be relaxed accurately, approximating the molecular motion of the actual polymer material.

For the calculation of such structural relaxation, COGNAC is used, which is included in a soft material comprehensive simulator (J-OCTA) produced by JSOL Ltd.

Next, the computer 1 judges whether the initial arrangement of the filler model 35 and the coarse-grained model 36 has been sufficiently relaxed or not. (step S38)

If the initial arrangement of the filler model 35 and the coarse-grained model 36 is judged in the step S38 as being sufficiently relaxed ("Y" in the step S38), the next step S4 is carried out.

On the other hand, if the initial arrangement of the filler model 35 and the coarse-grained model 36 is judged as being not sufficiently relaxed ("N" in the step S38 ), then a unit time is advanced (step S39), and the step S37 and step S38 are performed again.

Thereby, in this embodiment, the equilibrium state of the filler model 35 and the coarse-grained model 36 (a state in which the structure is relaxed) can be reliably calculated. Therefore, in the model defining step S3, since the high polymer material models 26 are accurately defined, the accuracy of the undermentioned deformation simulation can be improved.

Next, as shown in Figure 4, the computer 1 performs the deformation simulation based on the polymer material model 26 (step S4).

In the step S4, according to the uniaxial tensile test commonly performed with respect to the polymer material 2 (shown in Figurer 2), the polymer material model 26 (shown in Figure 10) is elongated in one direction (for example, 0% to 20% in the y-axis direction), and a physical quantity (for example, stress - strain curve) of the polymer material model 26 is calculated. Such physical quantity of the polymer material model 26 is stored in the computer 1.

In the present embodiment, since the filler model 35 and the coarse-grained model 36 are modeled independently, it is possible to simulate a large deformation such that a void is generated in the polymer material 2 (as shown in Figure 2) by the movement of the filler particle models 39 and the coarse-grained particle models 41 due to the deformation of the polymer material model 26.

On the other hand, in a finite element model which is commonly used for a long time in the simulations of the materials, and in which the adjacent elements share common nodes, it is impossible in principle to express it as being possible to generate voids. In addition, if the element is crushed at the time of large deformation, the finite element model will not satisfy the Courant condition, and the calculation will be failed.

Thus, in this embodiment, the behavior during large deformation of the polymer material 2 can be expressed with a high degree of accuracy.

Moreover, in the present embodiment, since the filler model 35 and the coarse-grained model 36 are defined based on the filler portion 27 and the polymer material portion 28 discriminated from the actual polymer material 2 (shown in Figure 2), the highly accurate polymer material model 26 can be defined. Thus, in this embodiment, the behavior of the polymer material 2 during large deformation can be expressed with a high degree of accuracy.

Incidentally, the method for deforming the polymer material model 26 is not limited to the method as described above. For example, it may be a method such that the polymer material model 26 is, after subjected to an initial elongation of 10%, deformed by applying periodical strain of +/- 1%, or a method such that the polymer material model 26 is subjected to compressive or shear deformation.

Next, as shown in Figure 4, the computer 1 judges whether the physical quantity of the polymer material model 26 is within a predetermined allowable range or not (step S5).

If the physical quantity of the polymer material model 26 is judged in the step S5 as being within the allowable range ("Y" in the step S5), then the polymer material 2 is produced based on the polymer material model 26 (step S6). On the other hand, if the physical quantity of the polymer material model 26 is judged as being outside the allowable range ("N" in the step S5), then the compounding of the filler 3 is changed (step S7), and the step S1 to step S5 are carried out again.

Thus, in the simulation method in the present embodiment, the polymer material 2 having the physical quantity within the allowable range can be produced.

In the micro region selecting step S32 in the present embodiment, the micro region 31 is defined at an arbitrary position in the three-dimensional structure 22 as shown in Figure 8, but it is not limited thereto.

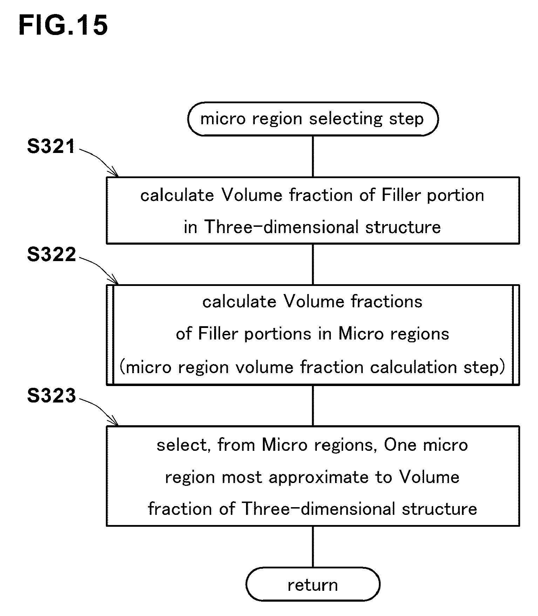

For example, in the micro region selecting step S32, the micro region 31 may be defined based on the volume fraction of the filler portion 27 in the three-dimensional structure 22. Figure 15 is a flowchart showing an example of the processing procedure of the micro region selecting step S32 in another embodiment of the present invention.

In the micro region selection step S32 in this embodiment, first, the volume fraction of the filler portion 27 in the three-dimensional structure 22 (shown in Figure 8) is computed (step S321).

The volume fraction $\phi b$ of the filler portion 27 in the three-dimensional structure 22 is determined based on the following equation (3).

$$\phi b = Vb / Va \ldots (3)$$

where,

    $\phi b$: the volume fraction of the filler portion in the three-dimensional structure of the polymer material
    Va: the volume (mm$^3$) of the three-dimensional structure of the polymer material
    Vb: the volume (mm$^3$) of the filler part in the three-dimensional structure of the polymer material

As shown in Figure 8, the volume Va of the three-dimensional structure of the polymer material is the volume of the whole area of the three-dimensional structure 22. The volume Vb of filler portion in the three-dimensional structure is the volume of all of the filler portions 27 disposed in the three-dimensional structure 22. The volume Vb of filler portion can be readily calculated by the computer 1 based on the filler portions 27 discriminated by the image processing.

Then, by dividing the volume Vb of filler portion by the volume Va of the three-dimensional structure, the volume fraction $\phi b$ of the filler portion in the three-dimensional structure is obtained.

Such volume fraction $\phi b$ is stored in the computer 1.

Next, in a plurality of the micro regions 31 having different positions in the three-dimensional structure 22, the volume fraction of the filler portion 27 in each micro region 31 is calculated (micro region volume fraction calculation step S322).

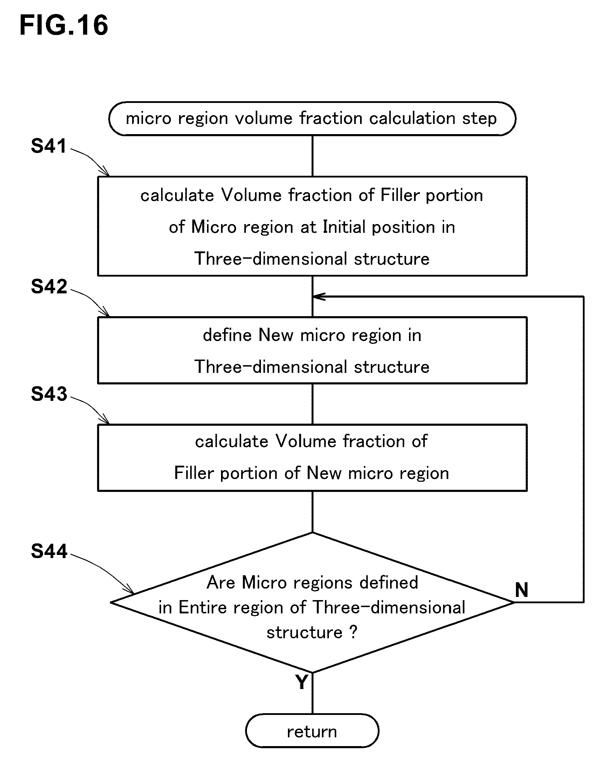

Figure 16 is a flowchart showing an example of the processing procedure of the micro region volume fraction calculation step S322.

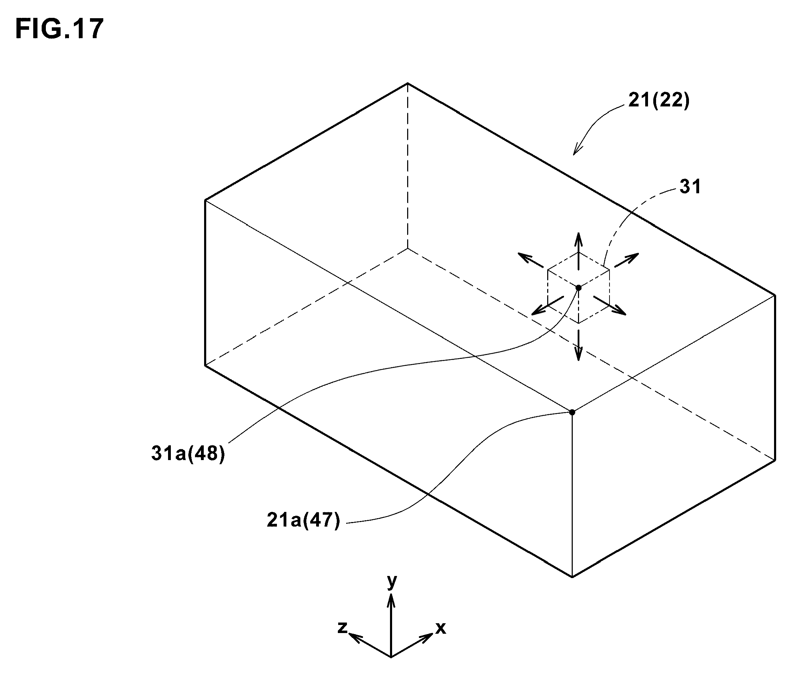

Figure 17 is a perspective view showing a micro region 31 in the three-dimensional structure 22.

In Figure 17, the filler portion 27 and the polymer material portion 28 shown in Figure 8 are omitted.

In the micro region volume fraction calculation step S322, first, the volume fraction of the filler portion 27 (shown in Figure 8) in the micro region 31 is calculated at the initial position at which the micro region 31 is initially arranged in the three-dimensional structure 22 (step S41).

The initial position can be set arbitrarily.

The initial position in this embodiment is, for example, set to a position at which a reference point 47 defined by a vertex 21a of the three-dimensional structure 22 and a reference point 48 defined by one of the vertices 31a of the micro region 31 coincide with other.

The volume fraction $\phi d$ of the filler portion 27 in the micro region 31 is determined based on the following equation (4).

$$\phi d = Vd / Vc \quad \cdots \quad (4)$$

where,

$\phi d$: the volume fraction of the filler portion in the micro region
Vc: the volume ($nm^3$) of the micro region
Vd: the volume ($nm^3$) of filler portion in the micro region

The volume Vc of the micro region is the volume of the entire region of the micro region 31.

The volume Vd of filler portion in the micro region is the volume of all of the filler portions 27 disposed in the micro region 31 (shown in Figure 8).

The volume Vd of filler portion can be calculated by the computer 1 based on the filler portions 27 disposed in the micro region 31, of the filler portions 27 of the three-dimensional structure 22.

Then, by dividing the volume Vd of filler portion in the micro region by the volume Vc of the micro region, the volume fraction $\phi d$ of the filler in the micro region is obtained. Such volume fraction $\phi d$ is stored in the computer 1.

Then, in the three-dimensional structure 22, a new micro region 31 is defined (step S42), and the volume fraction $\phi d$ of the filler portion 27 in the new micro region 31 is calculated (step S43).

The volume fraction $\phi d$ of the filler part in this new micro region 31 is stored in the computer 1.

In the step S42, the new micro region 31 is defined at a position different from the previously defined micro region 31. In the step S42, in the three-dimensional structure 22, for example, by moving the previously selected micro region 31 along the x-axis direction, y-axis direction or z-axis direction, the new micro region 31 is defined.

Incidentally, the distance (not shown) to move the micro region 31 can be set arbitrarily. The distance in this embodiment is desirably set to 1nm to 100nm.

Thus, in the three-dimensional structure 22, the micro regions 31 can be evenly defined.

Next, it is judged if the micro regions 31 are defined in the entire region in the three-dimensional structure 22 (step S44).

If the micro regions 31 are judged in the step S44 as being defined in the entire region in the three-dimensional structure 22, ("Y" in the step S44), then the next step S323 is performed. On the other hand, if the micro regions 31 are judged as being not defined ("N" in the step S44), then the step S42 and step S43 are performed again.

Thus, in the micro region volume fraction calculation step S322, the volume fraction $\phi d$ of filler portion in the micro region 31 can be calculated in the entire region in the three-dimensional structure 22.

Next, one micro region 31 is selected from a plurality of the micro regions 31 (step S323).

In the step S323, among a plurality of the micro regions 31, there is selected such a micro region 31 that the volume fraction $\phi d$ of the filler portion 27 in the micro region 31 is most approximate to the volume fraction $\phi b$ of the filler portion 27 in the three-dimensional structure 22.

The selected micro region 31 is stored in the computer 1. Then, based on the selected micro region 31, the polymer material model 26 is defined in the steps subsequent to the step S33 shown in Figure 9.

Thus, in this embodiment, for example, it can be avoided that the polymer material model 26 is defined based on the micro region 31 having the volume fraction $\phi d$ significantly different from the volume fraction $\phi b$ of the filler portion 27 of the three-dimensional structure 22, therefore the simulation accuracy can be improved.

In this embodiment, in the step S322, after the volume fraction $\phi d$ of filler portion 27 is calculated in a plurality of the micro regions 31, the volume fraction $\phi d$ of each micro region 31 is compared with the volume fraction $\phi b$ of the filler portion 27 in the three-dimensional structure 22, but it is not limited thereto.

For example, it may be possible to employ a method such that, every time the micro region 31 is defined, the volume fraction $\phi d$ of the micro region 31 and the volume fraction $\phi b$ of the three-dimensional structure 22 is compared, and the micro region 31 most closest to the volume fraction $\phi b$ of the three-dimensional structure 22 is selected. According to this method, it is not necessary to store the volume fraction $\phi d$ of all of the micro regions 31, therefore, the amount of data can be reduced.

while detailed description has been made of an especially preferable embodiment of the present invention, the present invention can be embodied in various forms without being limited to the illustrated embodiment

working Examples

Polymer material having the following composition was prepared. From the polymer material, a sample having a thickness of 500nm was made by the use of the following microtome (Experiment Example).
Based on the following specifications, the uniaxial tensile test was performed on the sample, and the mean absolute deviation of the stress-strain curve was obtained. Further, using coefficients obtained by fitting the autocorrelation function of the three-dimensional density distribution of the filler, to the power function by the method of least squares, a fractal dimension indicating the extent of the aggregate structure of the filler contained in the polymer material, was obtained.

According to the procedures shown in Figure 4 and Figure 9, the three-dimensional structure of the polymer material was constructed based on electron beam transmission images of the polymer material taken with a scanning transmission electron microscope.
Then, based on the three-dimensional structure of the polymer material, the polymer material model was defined (working example 1, working example 2).

In the micro region selecting step of the working example 1, the micro region defined at an arbitrary position of the three-dimensional structure was selected.

In the micro region selecting step of the working example 2, in a plurality of the micro regions defined at different positions in the three-dimensional structure of the polymer material, the micro region closest to the volume fraction of the filler portion in the three-dimensional structure was selected according to the procedures shown in Figure 15 and Figure 16.

For comparison, without using the three-dimensional structure of the polymer material, a plurality of filler models were arranged in a virtual space at regular intervals, and a plurality of coarse-grained models were arranged around the filler models (Comparative Example 1).
Further, based on the finite element method, a polymer material model was defined from the three-dimensional structure of the polymer material (Comparative Example 2).

Then, using the respective polymer material models working Example 1, working Example 2, Comparative Example 1 and Comparative Example 2, the deformation calculation based on the uniaxial tensile test was made, and the mean absolute deviation of the stress - strain curve was obtained.
Further, for each of the polymer material models working Example 1, working Example 2, Comparative Example 1 and Comparative Example 2, a fractal dimension indicating the extent of the aggregate structure of the filler models included in the polymer material model was obtained.

Each of the mean absolute deviations of working Examples 1 to Comparative Example 2 is indicated by an index based on Experimental Example being 1.0.
when each mean absolute deviation became closer to 1.0, the behavior of the polymer material during large deformation can be expressed with a high degree of accuracy.
Further, when the fractal dimensions of working Examples 1 to Comparative Example 2 became closer to the value of the fractal dimension of experimental example, the fillers blended in the polymer material can be expressed with a high degree of accuracy. incidentally, the numerical values of the potentials were set as described in this specification, and other common specifications are as follows.
The results are shown in Table 1.

Composition of the polymer material:

styrene-butadiene rubber (SBR): 100 parts by mass
silica: 50 parts by mass
sulfur: 1.5 parts by mass
vulcanization accelerator CZ: 1 part by mass
vulcanization accelerator DPG: 1 part by mass

Details of Composition:

styrene-butadiene rubber (SBR): Asahi Kasei chemicals Co., Ltd. E15
silica: ultrasil VN3 of Degussa Co., Ltd.
sulfur: powdered sulfur of Karuizawa sulfur Ltd.
vulcanization accelerator CZ: Nocceler CZ manufactured by ouchi Shinko chemical industrial Ltd.

vulcanization accelerator DPG: Nocceler D manufactured by ouchi Shinko chemical industrial Ltd.
scanning transmission electron microscope: JEM2100F (accelerating voltage 200 kv)
microtome: ultramicrotome EM VC6 manufactured by LEICA Co.
virtual space (cubic) :

length L1 of one side: 158nm (240σ)

filler model:

number of pieces arranged in the virtual space: 420 pieces

total number of filler particle models: 2.52 million pieces

coarse-grained model:

number of pieces arranged in the virtual space: 11500

number of coarse-grained particle models making up one coarse-grained model: 1000

uniaxial tensile test for the polymer material model:

deformation: 500% in the y-axis direction

[table 1]

|  | Experimental example | Comparative example 1 | Comparative example 2 | working example 1 | working example 2 |
|---|---|---|---|---|---|
| fractal dimension | 2.8 | 3.0 | 2.8 | 2.8 | 2.8 |
| mean absolute deviation of stress-strain curve | 1.00 | 0.80 | 0.50 | 0.90 | 0.95 |

Form the test results, it was confirmed that, in comparison with the polymer material models of Comparative Example 1 and Comparative Example 2, the polymer material models of working Example 1 and working Example 2 can approximate the mean absolute deviation and fractal dimension of Experimental Example. Therefore, it was confirmed that, in the simulation method of working Example 1 and working Example 2 can accurately express the behavior during large deformation of the polymer material.
Further, it was confirmed that, in comparison with the polymer material model of working Example 1, the polymer material model of working Example 2 can approximate the mean absolute deviation of Experimental example.

**Claims**

1. A simulation method for a polymer material which is a simulation method for calculating deformation of the polymer material containing a filler by the use of a computer, comprising
an imaging step of acquiring electron beam transmission images of the polymer material by the use of a scanning transmission electron microscope,
a step in which the computer constructs a three-dimensional image of the polymer material by a tomographic method based on the electron beam transmission images,
a model defining step in which the computer defines a polymer material model based on the three-dimensional image of the polymer material, and
a step in which the computer performs a deformation simulation based on the polymer material model,
and **characterized in that** the model defining step compresses
a step of constructing a three-dimensional structure of the polymer material in which a filler portion where the filler is arranged and a polymer material portion around the filler portion are discriminated based on the three-dimensional image of the polymer material,
a filler model arranging step of arranging, in the filler portion, at least one filler model obtained by modeling the filler by using a plurality of filler particle models and a coupling chain model coupling between the adjacent filler particle models,

a coarse-grained model arranging step of arranging, in the polymer material portion, at least one coarse-grained model obtained by modeling a macromolecular chain of the polymer material by using a plurality of coarse-grained particle models and a coupling chain model coupling between the adjacent coarse-grained particle models, and

a step in which the computer calculates a structural relaxation based on a molecular dynamics calculation by using the filler model and the coarse-grained model.

2. The simulation method for a polymer material as set forth in claim 1, which further comprises a micro region selecting step in which the computer selects a micro region partitioned in the three-dimensional structure of the polymer material and having a predetermined size, and

in the filler model arranging step, the filler model is arranged in the filler portion in the micro region, and

in the coarse-grained model arranging step, the coarse-grained model is arranged in the polymer material portion in the micro region.

3. The simulation method for a polymer material as set forth in claim 2, wherein the micro region selecting step comprises

a step of calculating the volume fraction of the filler portion in the three-dimensional structure of the polymer material,

a step of calculating the volume fraction of the filler portion in each micro region of a plurality of the micro regions partitioned at different positions in the three-dimensional structure of the polymer material,

a step of selecting, among a plurality of the micro regions, the micro region whose filler portion has the volume fraction mostly approximating the volume fraction of the filler portion in the three-dimensional structure of the polymer material.

4. The simulation method for a polymer material as set forth in any one of claims 1 to 3, wherein the filler particle models of the filler model are arranged in a face-centered cubic lattice.

5. The simulation method for a polymer material as set forth in any one of claims 1 to 4, wherein the coupling chain model of the filler model is defined according to a bond function or a particle distance restricting method.

**FIG.1**

# FIG.2

**FIG.3**

# FIG.4

```
                        ( start )
                           │
S1                         │◄─────────────────────────────┐
   ┌───────────────────────────────────────────┐          │
   │   obtain Electron beam transmission        │          │
   │      images of Polymer material            │          │
   │         (imaging step)                     │          │
   └───────────────────────────────────────────┘          │
S2                         │                               │
   ┌───────────────────────────────────────────┐          │
   │   construct Three-dimensional image        │          │
   │        of Polymer material                 │          │
   └───────────────────────────────────────────┘          │
S3                         │                               │
   ┌───────────────────────────────────────────┐          │
   │   define Polymer material model based      │          │
   │       on Three-dimensional image           │          │
   │          of Polymer material               │          │
   │        (model defining step)               │          │
   └───────────────────────────────────────────┘          │
S4                         │                               │
   ┌───────────────────────────────────────────┐          │
   │   perform Deformation simulation based     │          │
   │         on Polymer material model          │          │
   └───────────────────────────────────────────┘          │
                           │                            S7 │
                           │               ┌───────────────────────────┐
                           │               │ change Compounding of Filler │
S5                        ╱ ╲              └───────────────────────────┘
                       ╱       ╲                        ▲
                    ╱  Is Physical  ╲   N               │
                  ╱ quantity of Polymer ╲───────────────┘
                  ╲ material model within ╱
                    ╲  allowable range ? ╱
                       ╲       ╱
                          ╲ ╱
                           │ Y
S6                         │
   ┌───────────────────────────────────────────┐
   │   produce Polymer material based on        │
   │         Polymer material model             │
   └───────────────────────────────────────────┘
                           │
                        (  end  )
```

**FIG.5**

# FIG.6

**FIG.7**

**(a)**

**(b)**

**FIG.8**

722nm × 1191nm × 500nm

EP 3 062 092 A1

# FIG.9

**S31**

( model defining step )

construct Three-dimensional structure in which Filler portion and Polymer material portion are discriminated based on Three-dimensional image of Polymer material

**S32**

select Micro region defined in Three-dimensional structure (micro region selecting step)

**S33**

dispose Filler model in Filler portion in Micro region

**S34**

dispose Coarse-grained model in Polymer material portion in Micro region

**S35**

define Potential between Coarse-grained particle models

**S36**

define Potential between Filler particle models and between Coarse-grained particle model and Filler particle model

**S37**

perform Structural relaxation based on Molecular dynamics

**S38**

Has Initial arrangement been sufficiently relaxed ?  **N**

**S39**

advance Unit time

**Y**

( return )

# FIG.10

# FIG.11

EP 3 062 092 A1

## FIG.12

# FIG.13

# FIG.14

EP 3 062 092 A1

# FIG.15

micro region selecting step

S321

calculate Volume fraction of Filler portion
in Three-dimensional structure

S322

calculate Volume fractions
of Filler portions in Micro regions
(micro region volume fraction calculation step)

S323

select, from Micro regions, One micro
region most approximate to Volume
fraction of Three-dimensional structure

return

# FIG.16

micro region volume fraction calculation step

**S41**

calculate Volume fraction of Filler portion
of Micro region at Initial position in
Three-dimensional structure

**S42**

define New micro region in
Three-dimensional structure

**S43**

calculate Volume fraction of
Filler portion of New micro region

**S44**

Are Micro regions defined
in Entire region of Three-dimensional
structure ?

**N**

**Y**

return

# FIG.17

EP 3 062 092 A1

## INTERNATIONAL SEARCH REPORT

| International application No. |
| --- |
| PCT/JP2014/072318 |

| A. CLASSIFICATION OF SUBJECT MATTER |
| --- |
| *G01N23/04*(2006.01)i |

According to International Patent Classification (IPC) or to both national classification and IPC

| B. FIELDS SEARCHED |
| --- |

Minimum documentation searched (classification system followed by classification symbols)
G01N23/00-G01N23/227

Documentation searched other than minimum documentation to the extent that such documents are included in the fields searched

| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)
JSTPlus(JDreamIII)

| C. DOCUMENTS CONSIDERED TO BE RELEVANT |
| --- |

| Category* | Citation of document, with indication, where appropriate, of the relevant passages | Relevant to claim No. |
| --- | --- | --- |
| Y | Hiroyuki TAKAHASHI et al., "Viscoelastic Analysis of Filled rubber by Molecular Dynamics Simulation 2", Dai 24 Kai Elastomer Toronkai Koen Yoshishu, 29 November 2012 (29.11.2012), pages 29 to 30, * entire text, all drawings * | 1-5 |
| Y | JP 2013-061877 A (Sumitomo Rubber Industries, Ltd.), 04 April 2013 (04.04.2013), paragraphs [0013], [0016], [0031] to [0034], [0041] to [0043], [0052]; fig. 8, 10 | 1-5 |
| Y | Yoshihiro TOMITA, "Modeling and Simulation of Viscoelastic Deformation Behavior of Rubber Containing Fillers", Journal of the Society of Rubber Industry, Japan, vol.82, no.11, 2009.11, pages 464 to 471 * fig. 8 to 9, 12 to 13 * | 3 |

| ☒ Further documents are listed in the continuation of Box C. | ☒ See patent family annex. |
| --- | --- |

| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 03 October, 2014 (03.10.14) | 21 October, 2014 (21.10.14) |

| Name and mailing address of the ISA/ Japanese Patent Office | Authorized officer |
| --- | --- |
| Facsimile No. | Telephone No. |

Form PCT/ISA/210 (second sheet) (July 2009)

## INTERNATIONAL SEARCH REPORT

International application No.

PCT/JP2014/072318

C (Continuation).　DOCUMENTS CONSIDERED TO BE RELEVANT

| Category* | Citation of document, with indication, where appropriate, of the relevant passages | Relevant to claim No. |
|---|---|---|
| Y | GUSKOS,N. et.al. "In situ synthesis, morphology and magnetic properties of poly(ether-ester) multiblock copolymer/carbon-covered nickel nanosystems", JOURNAL OF NON-CRYSTALLINE SOLIDS, Volume 356, Issues 37-40, 2010.08.15, Pages 1893-1901 * Abstract, Figure 6 * | 4 |
| A | JP 2013-186746 A  (Sumitomo Rubber Industries, Ltd.), 19 September 2013 (19.09.2013), fig. 3 to 11 | 1 |
| A | RIGGLEMAN,R.A. et.al. "Entanglement network in nanoparticle reinforced polymers", THE JOURNAL OF CHEMICAL PHYSICS, Volume 130, Number 24, 2009.06.29, Article 244903, 6 Pages * Sections II-III * | 1 |
| A | JP 2011-069781 A  (Bridgestone Corp.), 07 April 2011 (07.04.2011), paragraphs [0001] to [0005] | 1 |

Form PCT/ISA/210 (continuation of second sheet) (July 2009)

33

INTERNATIONAL SEARCH REPORT
Information on patent family members

International application No.

PCT/JP2014/072318

| | | | |
|---|---|---|---|
| JP 2013-061877 A | 2013.04.04 | CN 102954975 A | 2013.03.06 |
| | | EP 2562534 A2 | 2013.02.27 |
| | | EP 2562534 A3 | 2014.05.21 |
| | | JP 2013-044607 A | 2013.03.04 |
| | | JP 2013-054578 A | 2013.03.21 |
| | | US 2013/051656 A1 | 2013.02.28 |
| JP 2013-186746 A | 2013.09.19 | JP 5427260 B2 | 2014.02.26 |
| | | CN 103310034 A | 2013.09.18 |
| | | EP 2642416 A2 | 2013.09.25 |
| | | US 2013/238302 A1 | 2013.09.12 |
| JP 2011-069781 A | 2011.04.07 | JP 5269732 B2 | 2013.08.21 |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2013057638 A

### Non-patent literature cited in the description

- **KURT KREMER ; GARY S. GREST.** Dynamics of entangled linear polymer melts: A molecular-dynamics simulation. *J Chem Phys,* 15 April 1990, vol. 92 (8

- Chain Dimension and Entanglement Spacings. **L,J.FETTERS ; D.J.LOHSE ; R.H.COLBY.** Physical Properties of Polymers Handbook. 448